# EUROPÄISCHE PATENTSCHRIFT

(11) **EP 1 081 125 B1**
(45) Veröffentlichungstag und Bekanntmachung des Hinweises auf die Patenterteilung: **11.06.2003**
(21) Anmeldenummer: 00117813.6
(22) Anmeldetag: 18.08.2000
(51) Int. Cl.: C07C 67/08, C07C 69/54

(54) **Verfahren zur Herstellung von (Meth)acrylsäureestern**
Process for the preparation of (meth)acrylic acid esters
Procédé de préparation d'esters de l'acide (méth)acrylique

(30) Priorität: 30.08.1999 DE 19941136
(43) Veröffentlichungstag der Anmeldung: 07.03.2001
(73) Patentinhaber: BASF AKTIENGESELLSCHAFT, 67056 Ludwigshafen (DE)
(72) Erfinder: Leube, Hartmann F., 67061 Ludwigshafen (DE); Leidinger, Kurt, 67117 Limburgerhof (DE); Geisendörfer, Matthias, 67435 Neustadt (DE); Beck, Erich, 68526 Ladenburg (DE)
(74) Vertreter: Kinzebach, Werner, Dr.

(56) Entgegenhaltungen:
- EP-A- 0 989 108
- DE-A- 3 316 593
- DE-A- 3 704 098
- DE-A- 3 836 370

## Beschreibung

Die Erfindung betrifft ein verbessertes Verfahren zur Herstellung von (Meth)acrylsäureestern durch Veresterung einer hochsiedenden Verbindung mit einer oder mehreren alkoholischen Hydroxylgruppen mit (Meth)acrylsäure in Gegenwart eines sauren Veresterungskatalysators und eines Schleppmittels unter Austragen des Reaktionswassers und temperaturkontrollierter Rückführung des Schleppmittels in das Reaktionsgemisch.

Die Veresterung von hochsiedenden Verbindungen mit aliphatischen Hydroxylgruppen mit (Meth)acrylsäure hat durch die breite Anwendung der Veresterungsprodukte für strahlungshärtbare Lacke und Beschichtungen eine große industrielle Bedeutung bekommen. Hierzu werden in Batchverfahren Hydroxylverbindung, (Meth)acrylsäure, Katalysator, Stabilisator und Schleppmittel vorgelegt. Nach dem Erhitzen zum Sieden wird dann das während der Veresterungsreaktion gebildete Reaktionswasser als Azeotrop von Schleppmittel und Wasser abdestilliert. Das abdestillierte Azeotrop wird kondensiert und in einem nachgeschalteten Phasenscheider in Wasser und Schleppmittel getrennt. Das abgetrennte Schleppmittel wird dann zur Auskreisung von weiterem Reaktionswasser direkt in das Reaktionsgefäß zurückgeführt. Nach beendeter Veresterungsreaktion und beendeter Abscheidung von freigesetztem Reaktionswasser wird das Schleppmittel insgesamt aus dem Reaktionsgemisch durch Abdestillieren entfernt (vgl. z. B. DE-A-3316593, DE-A-3704098, DE-A-3836370, EP-A-646567). Bei dem konventionellen Veresterungsfahren entspricht die Siedetemperatur der Reaktionstemperatur und, bedingt durch die Wasserauskreisungsmethode, ist der Gehalt von Schleppmittel im Reaktionsgemisch konstant.

Die EP-A 0 989 108, die erst nach dem für die vorliegende Anmeldung maßgeblichen Zeitrang veröffentlicht wurde, offenbart die Veresterung eines Alkohols mit (Meth)acrylsäure, wobei die Reaktionstemperatur auf höchstens 130 °C gehalten wird und die Umlaufgeschwindigkeit des Schleppmittels während der Veresterung mindestens 0,5 Cyclen/Stunde beträgt.

Seit langem wünscht man die lange Dauer der Veresterungsgleichgewichtsreaktion zu verkürzen. Eine Erhöhung der Reaktionsgeschwindigkeit kann z. B. durch eine Erhöhung der Reaktionstemperatur erfolgen, die jedoch die Gefahr einer Polymerisation der ungesättigten Monomeren während der Veresterungsreaktion erhöht. Auch ist zu beachten, dass die meisten der üblichen Stabilisatorsysteme bei hohen Veresterungstemperaturen leicht zu Produktverfärbungen führen. Dies begrenzt die Wahl hoher Veresterungstemperaturen, weil entstehende Verfärbungen nur durch mühsame Maßnahmen wie Destillation, Auswaschen etc. entfernt werden können. So soll z. B. bei dem sonst vorteilhaften Inhibitorsystem aus Kupfer(II)chlorid und Hydrochinonmonomethylether zur Erzielung hochwertiger farbloser Produkte eine Veresterungstemperatur von ca. 105 °C nicht überschritten werden.

Die Temperatur der Reaktionsmischung ist eine Funktion des Siedepunktes des verwendeten Schleppmittels. Hochsiedende Schleppmittel wie Toluol haben den Vorteil, dass sie eine hohe Schleppwirkung für Wasser zeigen und zu verhältnismäßig hohen Gemischsiedetemperaturen führen, wodurch die Veresterungsgeschwindigkeit gesteigert werden kann. Nachteilig ist, dass die hochsiedenden Schleppmittel sich schlecht aus manchen Endprodukten abtrennen lassen. Zudem müssen sie in großen Konzentrationen eingesetzt werden, da sonst die resultierende Gemischsiedetemperatur zu hoch wird und Polymerisationsgefahr besteht. Niedrigsiedende Schleppmittel, wie z. B. Cyclohexan, haben den Nachteil einer geringeren Schleppwirkung und einer Senkung der Reaktionstemperatur und somit der Reaktionsgeschwindigkeit. Doch haben sie den Vorteil, dass sie in geringeren Konzentrationen eingesetzt werden können und sich leichter nach Reaktionsende entfernen lassen.

Die Siedetemperatur des Reaktionsgemisches ist aber auch eine Funktion der Gemischzusammensetzung. Da die Gemischzusammensetzung sich während der Reaktionsdauer durch den Umsatz von Hydroxylverbindung und Säure zum Ester verschiebt, steigt auch die Reaktionstemperatur während der Veresterung. Bei konstanter Schleppmittelmenge erfordert dies, die Veresterungsreaktion zu Beginn bei niedrigen Temperaturen und mit niedriger Reaktionsgeschwindigkeit durchzuführen, um nicht am Ende der Reaktion Temperaturen zu erreichen, bei denen Polymerisationsgefahr besteht. Es resultieren so wirtschaftlich unerwünscht lange Veresterungszeiten.

Im Prinzip ist es möglich, die Siedetemperatur durch Kontrolle des Druckes zu regulieren, was aber ein Arbeiten in speziellen aufwendigen drucksicheren Apparaturen erfordert.

Der vorliegenden Erfindung lag die Aufgabe zugrunde, ein Verfahren zur Herstellung von Estern der (Meth)acrylsäure mit hochsiedenden Alkoholverbindungen zur Verfügung zu stellen, bei dem eine gute gleichbleibenden Produktqualität bei einer möglichst gleichmäßigen optimalen Reaktionstemperatur erzielt wird. Eine weitere Aufgabe war, eine Erhöhung der Reaktionsgeschwindigkeit und somit eine Verkürzung der Reaktionszeit bei der Veresterungsreaktion zu erreichen.

Es wurde nun gefunden, dass die Aufgaben gelöst werden können mit einem Verfahren zur Herstellung von (Meth)acrylsäureestern durch Veresterung einer hochsiedenden Verbindung mit ein oder mehreren alkoholischen Hydroxylgruppen mit (Meth)acrylsäure in Gegenwart eines sauren Veresterungskatalysators, eines Schleppmittels für das Reaktionswasser und eines Polymerisationsinhibitors unter Erhitzen des Reaktionsgemisches zum Sieden und Austragen von Reaktionswasser mit Hilfe des Schleppmittels und Zuführung von Schleppmittel zum Reaktionsgemisch während der Veresterungsreaktion, bei dem das Schleppmittel dem Reaktionsgemisch während mehr als 70 % der Zeitdauer der Veresterungsreaktion diskontinuierlich temperaturkontrolliert so zudosiert wird, dass die Siedetemperatur des Reaktionsgemisches im Bereich von ± 2 °C konstant gehalten wird, wobei das Schleppmittel nachdosiert wird, wenn das Volumen des Reaktionsgemisches durch ausgetragenes Reaktionswasser abnimmt.

Beim erfindungsgemäßen Verfahren wird also der übliche Zwangsrücklauf von Schleppmittel aus dem Phasenscheider im Rahmen der Auskreisung unterbunden. Stattdessen wird das Schleppmittel temperaturkontrolliert bzw. von der jeweiligen Temperatur des Reaktionsgemisches gesteuert so in das Reaktionsgemisch dosiert, dass über den Siedepunkt des Reaktionsgemisches während der überwiegenden Dauer (d. h. während mehr als 70 %, bevorzugt mehr als 80 % oder 90 % der gesamten Dauer) und bevorzugt während der im Wesentlichen gesamten Dauer der Veresterungsreaktion eine gleichmäßige Innentemperatur der Reaktionsmischung resultiert. Bevorzugt sollen die Schwankungen der Siedetemperaturen, von anfänglichen Einstellungsschwankungen abgesehen, nicht größer als ± 2 °C betragen. Auf diese Weise kann während der gesamten Veresterungsreaktion bei einer gleichmäßig optimalen Reaktionstemperatur gearbeitet werden, was, wie gesagt, zu einer gleichmäßig guten Produktqualität und zu einer Verkürzung der Reaktionszeiten der Veresterung führt.

Bevorzugt liegt die Siedetemperatur des Reaktionsgemisches einschließlich Schleppmittel im Bereich von 70 bis 110 °C und besonders bevorzugt im Bereich von 75 bis 105 °C, wobei niedrig siedende Schleppmittel verwandt werden. Die Siedetemperatur des Reaktionsgemisches liegt dabei vorzugsweise mehr als 5 °C, insbesondere mehr als 10 °C, höher als der Siedepunkt des reinen Schleppmittels (bei gleichem Druck).

Ein weiterer Vorteil des erfindungsgemäßen Verfahrens ist, dass der Reaktionskessel für die Veresterung von Anfang an höher mit dem Reaktionsgemisch befüllt werden kann, da zu Beginn der Veresterungsreaktion weniger Volumen für das Schleppmittel erforderlich ist. Wenn das Volumen des Reaktionsgemisches durch ausgetragenes Reaktionswasser abnimmt, wird das Schleppmittel nachdosiert.

Als Schleppmittel kommen insbesondere aliphatische und cycloaliphatische gesättigte Kohlenwasserstoffe in Betracht, die mit Wasser ein Azeotrop bilden können und eine Siedetemperatur im Bereich von 40 bis 120 °C und insbesondere im Bereich von 70 bis 110 °C aufweisen. Geeignete niedrigsiedende Schleppmittel sind z. B. n-Hexan, n-Heptan, Cyclohexan und Methylcyclohexan, Spezialbenzine und handelsübliche aliphatische Kohlenwasserstoffgemische mit Siedetemperaturen in den angegebenen Temperaturbereichen. Als Schleppmittel bevorzugt ist Cyclohexan. Im Allgemeinen werden die Schleppmittel in einer Menge von 1 bis 80 und insbesondere 1 bis 35 Gew.-%, bezogen auf die Summe der Mengen von Hydroxylverbindung und (Meth)acrylsäure, verwendet.

Als hochsiedende, d. h. zumindest höher als die Siedetemperatur des verwendeten Schleppmittels siedende Verbindungen mit einer oder mehreren alkoholischen Hydroxylgruppen kommen neben hochsiedenden einwertigen Alkoholen wie Laurylalkohol oder 2-Ethylhexylalkohol insbesondere Polyole mit 2 bis 6 alkoholischen Hydroxylgruppen in Frage. Beispiele von Polyolen mit alkoholischen Hydroxylgruppen, die insbesondere 2 bis 20 C-Atome haben, sind Ethylenglykol, Propylenglykol, 1,2-, 1,3- und 1,4-Butandiol, Neopentylglykol, 1,5-Pentandiol, 1,6-Hexandiol, Glyzerin, Trimethylolethan, Trimethylolpropan, Ditrimethylolpropan, Sorbit, Pentaerythrit und Dipentaerythrit. Sehr geeignet als Komponente mit alkoholischen Hydroxylgruppen sind auch Oligomere von C₂-C₄-Alkylenglykolen, die insbesondere 2 bis 4 Alkylenglykoleinheiten im Molekül enthalten, wie z. B. Diethylenglykol, Triethylenglykol, Dipropylenglykol oder Tripropylenglykol. Sehr geeignete Hydroxylverbindungen für die erfindungsgemäße Veresterung sind auch Alkoxylierungsprodukte von Alkoholen und insbesondere ethoxylierte und/oder propoxylierte mehrwertige Alkohole wie ethoxyliertes Trimethylolpropan, ethoxyliertes und/oder propoxyliertes Pentaerythrit. Allgemein enthalten solche alkoxylierte Produkte 1 bis 20 und bevorzugt 1 bis 10 Alkoxygruppen pro Polyolmolekül. Als Polyole können neben Polyetherpolyolen auch Polyesterpolyole, ethermodifizierte Polyesterpolyole, mehrere aliphatische Hydroxylgruppen aufweisende Polyepoxydharze oder entsprechende Polyurethane verwendet werden. Bevorzugt sind für die Veresterung flüssige bis viskose niedermolekulare und oligomere Verbindungen mit alkoholischen Hydroxylgruppen.

Als Säuren werden für die Veresterung Methacrylsäure, Acrylsäure oder Gemische dieser Säuren verwendet, wobei als Säure die Acrylsäure bevorzugt ist.

Für die Veresterungsreaktion werden die Säure- und Hydroxylgruppen enthaltenden Komponenten insbesondere in Mengen von 1 bis 1,5 mol (Meth)acrylsäure pro Hydroxylgruppe der Mono- oder Polyolkomponente eingesetzt. Jedoch kann die Säuremenge auch entsprechend herabgesetzt sein, wenn nur ein Teil der Hydroxylgruppen von Polyolen in Acrylate und/oder Methacrylate überführt werden soll.

Als saure Veresterungskatalysatoren kommen starke organische oder anorganische Säuren in Frage, die allgemein in Mengen von 0,1 bis 5 Gew.-%, bezogen auf die Summe der Mengen von Alkohol- und (Meth)acrylsäurekomponente, eingesetzt werden. Bevorzugte saure Veresterungskatalysatoren sind Schwefelsäure, p-Toluolsulfonsäure, Methansulfonsäure sowie stark saure Ionenaustauscher.

Zur Vermeidung einer Polymerisation von ungesättigten Monomeren wird die Veresterung der Hydroxylverbindungen mit (Meth)acrylsäure in Gegenwart geringer Mengen von Polymerisationsinhibitoren durchgeführt. Dabei handelt es sich um bekannte, zur Verhinderung einer thermischen Polymerisation verwendete organische und/oder anorganische Verbindungen wie z. B. Hydrochinon, Hydrochinonmonoalkylether, 2,6-Di-tert-butylphenol, Nitrosamine, Phenothiazin, Kupferverbindungen, wie Kupfer(II)chlorid, oder Kombinationen solcher Verbindungen. Die Polymerisationsinhibitoren werden dem Veresterungsgemisch in üblichen Mengen zugesetzt, im Allgemeinen in Mengen von 0,001 bis 5 und insbesondere 0,005 bis 1 Gew.-%, bezogen auf die Summe der Mengen an Hydroxylverbindung und (Meth)- acrylsäure.

Es hat sich als vorteilhaft erwiesen, dem Veresterungsgemisch vor der Veresterungsreaktion zusätzlich Farbstabilisatoren zuzusetzen, wie Unterphosphorige Säure (H₃PO₂), Triphenylphosphit oder eine Organophosphonsäure, wie 1-Hydroxy-ethan-1,1-diphosphonsäure, wobei die Menge dieser Zusätze im Allgemeinen 0,01 bis 3 Gew.-% beträgt, bezogen auf die Summe der Mengen der Hydroxylund (Meth)acrylsäurekomponente.

Nach Beendigung der Veresterungsreaktion, bei der ein Veresterungsgrad von mindestens 85 und insbesondere 90 bis 95 % erzielt werden soll, können die an sich bekannten oder üblichen Maßnahmen zur weiteren Aufarbeitung der Reaktionsprodukte durchgeführt werden. So kann dann das Schleppmittel und ggf. überschüssige (Meth)acrylsäure abdestilliert werden, bevorzugt bei 100 bis 120 °C bei einem Druck von 10 bis 150 mbar. Die sauren Veresterungskatalysatoren können neutralisiert werden, zugesetzte Inhibitoren bzw. Stabilisatoren können ausgewaschen oder gefällt werden und letzte Mengen an (Meth)acrylsäure können in Gegenwart geeigneter Katalysatoren mit Epoxiden umgesetzt werden. Die vorliegende Erfindung betrifft die Phase der Veresterungsreaktion der Hydroxylkomponente mit (Meth)acrylsäure und beschränkt somit in keiner Weise die dem Fachmann bekannte geeignete Aufarbeitung der erhaltenen Veresterungsprodukte nach bekannten bzw. üblichen Maßnahmen.

Die nachstehenden Beispiele sollen die vorliegende Erfindung weiter erläutern, jedoch nicht beschränken.

### Beispiel 1 (Vergleichsversuch)

2832 g propoxyliertes Trimethylolpropan ( 3 mol Propylenoxyd pro Mol Trimethylolpropan), 2249 g Acrylsäure, 221 g einer 65 gew.-%igen wässrigen Lösung von p-Toluolsulfonsäure als Veresterungskatalysator, 1663 g Cyclohexan als Schleppmittel, 1,2 g Kupfer(II)chlorid und 1 g Hydrochinonmonomethylether als Inhibitoren wurden in einem 7 Liter Rührgefäß aus Glas mit aufgesetztem Kondensator, Nachkondensator und Phasentrenngefäß vorgelegt. Das Reaktionsgemisch wurde zum Sieden erhitzt und das entstehende Dampfgemisch in Kondensator und Nachkondensator aus Glas vollständig kondensiert. Danach erfolgte eine Phasentrennung in dem Phasentrenngefäß. Das in der Veresterungsreaktion entstandene Reaktionswasser wurde ausgekreist und dessen entstandene Menge stündlich gemessen. Gleichzeitig wurde auch die erreichte Innentemperatur des Reaktionsgemisches (Ti) stündlich gemessen.

Es wurde nach konventioneller Verfahrensweise (stetiges Kreisen des im Ansatz vorhandenen Schleppmittels) das im Phasentrenngefäß abgetrennte Cyclohexan als Schleppmittel direkt in das Rührgefäß mit der Reaktionsmischung zurückgeführt. Die Reaktion wurde fortgeführt, bis sich im Phasentrenngefäß kein Wasser mehr abschied. Der erreichte Fortschritt der Veresterungsreaktion wurde anhand der Menge des abgetrennten Reaktionswassers bestimmt. Die stündlich gemessenen Innentemperaturen des Reaktionsgemisches (Ti) und erreichten Mengen an Reaktionswasser (H₂O) zeigt Tabelle 1. Bei ansteigenden Reaktionstemperaturen (78 bis 94 °C) wurden nach 13 Stunden 652 g, nach 11 Stunden 634 g Reaktionswasser abgeschieden.

**Tabelle 1**

| Beispiel 1 (Vergleichsversuch) - Stündlich gemessene Mengen an Reaktionswasser (H ₂ O) und Reaktorinnentemperaturen (Ti) | | | | | | | | | | | | | | |
|---|---|---|---|---|---|---|---|---|---|---|---|---|---|---|
| Zeit (h) | 0 | 1 | 2 | 3 | 4 | 5 | 6 | 7 | 8 | 9 | 10 | 11 | 12 | 13 |
| H ₂ O (g) | 0 | 72 | 233 | 386 | 454 | 523 | 576 | 589 | 600 | 602 | 616 | 634 | 645 | 652 |
| Ti (°C) | 78 | 79 | 81 | 86 | 88 | 89 | 91 | 92 | 92 | 92 | 93 | 93 | 94 | 94 |

### Beispiel 2 (erfindungsgemäß)

Es wurde wie in Beispiel 1 verfahren, jedoch wurde das kondensierte Cyclohexan als Schleppmittel nicht direkt aus dem Phasentrenngefäß in das Reaktionsrührgefäß zurückgeführt, sondern in ein Vorratsgefäß überführt. Getrennt wurde Cyclohexan in den Mengen in das Reaktionsrührgefäß dosiert, wie es zur Aufrechterhaltung der gewünschten Innentemperatur des Reaktionsgemisches von 95 °C während der Veresterungsreaktion erforderlich war. Die stündlich gemessenen Innentemperaturen (Ti) des Reaktionsgemisches sowie die erreichten Mengen an Reaktionswasser (H₂O) zeigt Tabelle 2. Es zeigte sich, dass bei einer konstanten Temperatur des Reaktionsgemisches von 94 bis 95 °C bereits nach 7 Stunden 634 g Reaktionswasser abgeschieden waren, d. h. die Veresterungsreaktion deutlich schneller verlief als in Beispiel 1 mit konventioneller Verfahrensweise.

**Tabelle 2**

| Beispiel 2 (erfindungsgemäß) - Stündlich gemessene Mengen an Reaktionswasser (H₂O) und Innentemperaturen des Reaktionsgemisches (Ti) | | | | | | | | |
|---|---|---|---|---|---|---|---|---|
| Zeit (h) | 0 | 1 | 2 | 3 | 4 | 5 | 6 | 7 |
| H₂O (g) | 0 | 324 | 492 | 598 | 625 | 629 | 629 | 634 |
| Ti (°C) | 95 | 94 | 95 | 95 | 95 | 95 | 95 | 95 |

### Beispiel 3 (Vergleichsversuch)

2077 g 1,6-Hexandiol, 3148 g Acrylsäure, 120 g einer 65 gew.-%igen wässrigen Lösung von p-Toluolsulfonsäure als Veresterungskatalysator, 1614 g Cyclohexan als Schleppmittel, 1,2 g Kupfer(II)chlorid und 1,5 g Hydrochinonmonomethylether als Inhibitoren wurden in einem 7 l Rührgefäß aus Glas vorgelegt. Dann wurde wie in Beispiel 1 angegeben verfahren, d. h. nach konventioneller Verfahrensweise das im Phasentrenngefäß abgetrennte Cyclohexan als Schleppmittel direkt in das Rührgefäß mit der Reaktionsmischung zurückgeführt. Die in stündlichen Zeitabständen gemessenen Innentemperaturen des Reaktionsmischung (Ti) sowie die abgeschiedenen Mengen an Reaktionswasser (H₂O) zeigt Tabelle 3.
Bei einer von 78 auf 101 °C steigenden Innentemperatur des Reaktionsgemisches Ti wurden nach 8 Stunden 730 g Reaktionswasser abgeschieden.

**Tabelle 3**

| Beispiel 3 (Vergleichsversuch) - Stündlich gemessene Mengen an Reaktionswasser (H₂O) und Innentemperaturen des Reaktionsgemisches (Ti) | | | | | | | | | |
|---|---|---|---|---|---|---|---|---|---|
| Zeit (h) | 0 | 1 | 2 | 3 | 4 | 5 | 6 | 7 | 8 |
| H₂O (g) | 0 | 173 | 391 | 484 | 489 | 597 | 721 | 728 | 730 |
| Ti (°C) | 78 | 80 | 84 | 89 | 96 | 100 | 101 | 101 | 101 |

### Beispiel 4 (erfindungsgemäß)

Das vorgelegte Reaktionsgemisch entsprach der in Beispiel 3 angegebenen Vorlage. Danach wurde jedoch wie in Beispiel 2 angegeben verfahren, d. h. das kondensierte Cyclohexan als Schleppmittel wurde nicht direkt aus dem Phasentrenngefäß in das Reaktionsgefäß zurückgeführt, sondern in ein Vorratsgefäß überführt. Getrennt wurde Cyclohexan in den Mengen in das Reaktionsgefäß dosiert, wie es zur Aufrechterhaltung der gewählten Innentemperatur des Reaktionsgemisches während der wesentlichen Dauer der fortschreitenden Veresterungsreaktion erforderlich war. Die stündlich gemessenen Innentemperaturen des Reaktionsmischung (Ti) sowie die erreichten Mengen an abgeschiedenen Reaktionswasser (H₂O) zeigt Tabelle 4. Es zeigte sich, dass bei nicht ansteigenden Innentemperaturen bei fortgeschrittener Veresterungsreaktion bereits nach 6 Stunden 730 g Reaktionswasser abgeschieden waren, d. h. eine schnellere Veresterungsreaktion erzielt werden konnte.

**Tabelle 4**

| Beispiel 4 (erfindungsgemäß) - Stündlich gemessene Mengen an Reaktionswasser (H₂O) und Innentemperaturen der Reaktionsmischung (Ti) | | | | | | | |
|---|---|---|---|---|---|---|---|
| Zeit (h) | 0 | 1 | 2 | 3 | 4 | 5 | 6 |
| H₂O (g) | 0 | 397 | 442 | 549 | 649 | 683 | 730 |
| Ti (°C) | 78 | 101 | 97 | 95 | 95 | 95 | 95 |

## Patentansprüche

1. Verfahren zur Herstellung von (Meth)acrylsäureestern durch Veresterung einer Verbindung mit einer oder mehreren alkoholischen Hydroxylgruppen mit (Meth)acrylsäure in Gegenwart eines sauren Veresterungskatalysators, eines Schleppmittels für das Reaktionswasser und eines Polymerisationsinhibitors unter Erhitzen des Reaktionsgemisches zum Sieden und Austragen von Reaktionswasser mit Hilfe des Schleppmittels sowie Zudosieren von Schleppmittel während der Veresterungsreaktion, wobei die Verbindung mit einer oder mehreren alkoholischen Hydroxylgruppen hoher als die Siedetemperatur des verwendeten Schleppmittels fiedet, **dadurch gekennzeichnet, dass** das Schleppmittel dem Reaktionsgemisch während mehr als 70 % der Zeitdauer der Veresterungsreaktion temperaturkontrolliert so zudosiert wird, dass die Siedetemperatur des Reaktionsgemisches im Bereich von ± 2 °C konstant gehalten wird, wobei das Schleppmittel nachdosiert wird, wenn das Volumen des Reaktionsgemisches durch ausgetragenes Reaktionswasser abnimmt.

2. Verfahren nach Anspruch 1, **dadurch gekennzeichnet, dass** die Siedetemperatur des Reaktionsgemisches im Bereich von 70 bis 110 °C liegt.

3. Verfahren nach Anspruch 1 oder 2, **dadurch gekennzeichnet, dass** die Siedetemperatur des Reaktionsgemisches im Bereich von 75 bis 105 °C liegt.

4. Verfahren nach einem der Ansprüche 1 bis 3, **dadurch gekennzeichnet, dass** das Schleppmittel ein Kohlenwasserstoff mit einer Siedetemperatur von 40 bis 120 °C ist.

5. Verfahren nach einem der Ansprüche 1 bis 4, **dadurch gekennzeichnet, dass** das Schleppmittel Cyclohexan ist.

6. Verfahren nach einem der Ansprüche 1 bis 5, **dadurch gekennzeichnet, dass** bei der Veresterung der Hydroxylverbindung mit Acryl- und/oder Methacrylsäure das Äquivalentverhältnis von Hydroxylgruppen zu Carboxylgruppen 1:1,0 bis 1:1,5 beträgt.

7. Verfahren nach einem der Ansprüche 1 bis 6, **dadurch gekennzeichnet, dass** als zu veresternde Hydroxylverbindung ein aliphatischer oder cycloaliphatischer Alkohol verwendet wird.

8. Verfahren nach einem der Ansprüche 1 bis 6, **dadurch gekennzeichnet, dass** als zu veresternde Hydroxylverbindung ein Oligomeres von Ethylenglykol und/oder Propylenglykol verwendet wird.

9. Verfahren nach einem der vorhergehenden Ansprüche, **dadurch gekennzeichnet, dass** die Siedetemperatur des Reaktionsgemisches mehr als 5 °C höher als der Siedepunkt des reinen Schleppmittels liegt.

## Claims

1. A process for the preparation of (meth)acrylates by esterifying a compound having one or more alcoholic hydroxyl groups with (meth)acrylic acid in the presence of an acidic esterification catalyst, of an entraining agent for the water of reaction and of a polymerization inhibitor while heating the reaction mixture to the boil and discharging the water of reaction with the aid of the entraining agent and metering in entraining agent during the esterification reaction, the compound having one or more alcoholic hydroxyl groups having a boiling point higher than the boiling point of the entraining agent used, wherein the entraining agent is metered into the reaction mixture under temperature control during more than 70% of the duration of the esterification reaction so that the boiling point of the reaction mixture is kept constant within ± 2°C, further entraining agent being metered in if the volume of the reaction mixture decreases owing to the discharged water of reaction.

2. A process as claimed in claim 1, wherein the boiling point of the reaction mixture is from 70 to 110°C.

3. A process as claimed in claim 1 or 2, wherein the boiling point of the reaction mixture is from 75 to 105°C.

4. A process as claimed in any of claims 1 to 3, wherein the entraining agent is a hydrocarbon having a boiling point of from 40 to 120°C.

5. A process as claimed in any of claims 1 to 4, wherein the entraining agent is cyclohexane.

6. A process as claimed in any of claims 1 to 5, wherein, in the esterification of the hydroxy compound with acrylic and/or methacrylic acid, the ratio of the number of equivalents of hydroxyl groups to that of carboxyl groups is from 1:1.0 to 1:1.5.

7. A process as claimed in any of claims 1 to 6, wherein an aliphatic or cycloaliphatic alcohol is used as the hydroxy compound to be esterified.

8. A process as claimed in any of claims 1 to 6, wherein an oligomer of ethylene glycol and/or propylene glycol is used as the hydroxy compound to be esterified.

9. A process as claimed in any of the preceding claims, wherein the boiling point of the reaction mixture is more than 5°C higher than the boiling point of the pure entraining agent.

## Revendications

1. Procédé pour la préparation d'esters d'acide (méth)acrylique par estérification d'un composé ayant un ou plusieurs groupes hydroxyle alcooliques avec de l'acide (méth)acrylique en présence d'un catalyseur d'estérification acide, d'un agent pour l'entraînement de l'eau de réaction et d'un inhibiteur de polymérisation, avec chauffage du mélange réactionnel à l'ébullition et élimination de l'eau de réaction au moyen du produit d'entraînement, ainsi qu'addition en quantité dosée de produit d'entraînement pendant la réaction d'estérification, tandis que le composé ayant un ou plusieurs groupes hydroxyle alcooliques bout au-dessus de la température d'ébullition du produit d'entraînement utilisé, **caractérisé par le fait que** l'agent d'entraînement est ajouté en quantité dosée au mélange réactionnel, avec réglage de température, pendant plus de 70 % de la durée de la réaction d'estérification, que la température d'ébullition du mélange réactionnel est maintenue constante dans l'intervalle de ± 2°C, tandis que l'agent d'entraînement est surdosé lorsque le volume du mélange réactionnel diminue sous l'effet de l'extraction de l'eau de réaction.

2. Procédé selon la revendication 1, **caractérisé par le fait que** la température d'ébullition du mélange réactionnel se situe dans l'intervalle de 70 à 110°C.

3. Procédé selon la revendication 1 ou 2, **caractérisé par le fait que** la température d'ébullition du mélange réactionnel se situe dans l'intervalle de 75 à 105°C.

4. Procédé selon l'une des revendications 1 à 3, **caractérisé par le fait que** l'agent d'entraînement est un hydrocarbure ayant une température d'ébullition de 40 à 120°C.

5. Procédé selon l'une des revendications 1 à 4, **caractérisé par le fait que** l'agent d'entraînement est le cyclohexane.

6. Procédé selon l'une des revendications 1 à 5, **caractérisé par le fait que** lors de l'estérification du composé hydroxylé avec de l'acide acrylique et/ou méthacrylique le rapport d'équivalents des groupes hydroxyle aux groupes carboxyle est de 1:1,0 à 1:1,5.

7. Procédé selon l'une des revendications 1 à 6, **caractérisé par le fait qu'**on utilise comme composé hydroxylé à estérifier un alcool aliphatique ou cycloaliphatique.

8. Procédé selon l'une des revendications 1 à 6, **caractérisé par le fait qu'**on utilise comme composé hydroxylé à estérifier un oligomère d'éthylèneglycol et/ou de propylèneglycol.

9. Procédé selon l'une des revendications précédentes, **caractérisé par le fait que** la température d'ébullition du mélange réactionnel se situe plus de 5°C au-dessus du point d'ébullition de l'agent d'entraînement pur.
